# EUROPEAN PATENT SPECIFICATION

(11) **EP 4 433 000 B1**
(45) Date of publication and mention of the grant of the patent: **04.11.2026**
(21) Application number: 22800823.1
(22) Date of filing: 25.10.2022
(51) Int. Cl.: A61F 9/008

(54) **EVALUATING AND TREATING EYE FLOATERS**
BEWERTUNG UND BEHANDLUNG VON AUGENSCHWIMMERN
ÉVALUATION ET TRAITEMENT DE MYODÉSOPSIE

(30) Priority: 19.11.2021 US 202163281382 P
(43) Date of publication of application: 25.09.2024
(73) Proprietor: Alcon Inc., 1701 Fribourg (CH)
(72) Inventor: BOR, Zsolt, Lake Forest, California 92630 (US)
(74) Representative: Murgitroyd & Company
(86) International application number: PCT/IB2022/060255
(87) International publication number: WO 2023/089416

(56) References cited:
- DE-A1- 102019 007 147
- NICUSOR IFTIMIA ET AL: "Hybrid retinal imager using line-scanning laser ophthalmoscopy and spectral domain optical coherence tomography", OPTICS EXPRESS, vol. 14, no. 26, 22 December 2006 (2006-12-22), XP055018733, DOI: 10.1364/OE.14.012909
- MILSTON REBECCA ET AL: "Vitreous floaters: Etiology, diagnostics, and management", SURVEY OF OPHTHALMOLOGY, vol. 61, no. 2, 1 March 2016 (2016-03-01), pages 211 - 227, XP029439479, ISSN: 0039-6257, DOI: 10.1016/J.SURVOPHTHAL.2015.11.008

## Description

### TECHNICAL FIELD

The present disclosure relates generally to ophthalmic laser surgical systems, and more particularly to evaluating and treating eye floaters.

### BACKGROUND

In ophthalmic laser surgery, a surgeon may direct a laser beam into the eye to treat the eye. For example, in laser vitreolysis, a laser beam is directed into the vitreous to treat eye floaters. Eye floaters are clumps of collagen proteins that form in the vitreous. These clumps can disturb vision with moving shadows and distortions. The laser beam fragments the floaters to improve vision.

Reference is made to the DE 10 2019 007147 A1, Nicusor let al "Hybrid retinal imager using line-scanning laser ophthalmoscopy and spectral domain optical coherence tomography", Optics Express, vol. 14, no. 26, (2006-12-22) abstract, and Milston R et al "Vitreous floaters: Etiology, diagnostics, and management", Survey of Ophthalmology, SURVEY OF OPHTHALMOLOGY, vol. 61, no. 2, pages 211-227, ISSN: 0039-6257 which have been cited as exemplary of the background state of the art.

### BRIEF SUMMARY

The scope of the invention is in accordance with the appended claims. The specification includes description of related methods, not claimed in the appended claims, but provided to assist in understanding the invention.

In certain embodiments, an ophthalmic laser surgical system for treating a floater in an eye comprises a scanning laser ophthalmoscope (SLO) device, an interferometer device, a laser device, and an xy-scanner. The SLO device generates an SLO image of a floater shadow cast by the floater onto a retina of the eye, and provides an xy-location of the floater shadow, where the xy-location is related to the xy-scanner. The interferometer device provides a z-location of the floater, where the z-location is relative to the retina. The laser device generates a laser beam and includes a z-focusing component that focuses a focal point of the laser beam onto the z-location of the floater. The xy-scanner: receives an SLO beam from the SLO device and directs the SLO beam along an SLO beam path towards the xy-location of the floater shadow; and receives the laser beam from the laser device and directs the laser beam along the SLO beam path towards the xy-location of the floater shadow.

Embodiments may include none, one, some, or all of the following features:
The interferometer device comprises an optical coherence tomography (OCT) device.

The interferometer device comprises a swept source A-scan interferometer (SSASI) device.

The interferometer device comprises multiple reference arms, where each arm corresponds to a z-range of multiple z-ranges within the vitreous, where a z-range is relative to the retina.

The ophthalmic laser surgical system includes an xy-encoder that: detects an angular position of the xy-scanner, where the angular position corresponds to an xy-location expressed in encoder units; and reports the xy-location expressed in encoder units.

The laser device includes adaptive optics that minimize a spot size of the laser beam.

The laser device includes adaptive optics that maximize a feedback signal to optimize the laser beam.

The laser device includes an optical element that form the laser beam as a Bessel or Bessel-like long focal length beam.

The computer: performs image processing on the SLO image of the floater shadow cast onto the retina; evaluates the floater shadow to determine if the floater can cause a visual disturbance; and outputs results of the evaluation. The computer may determine if the floater shadow is cast onto a foveal region or a parafoveal region of the eye. The computer may determine if the floater shadow is larger than a critical shadow size. The computer may generate and output a report comprising the SLO image of the floater shadow cast onto the retina and educational information about the floater or a recommended treatment for the floater.

In certain arrangements of the specification, a method, not claimed in the appended claims, for treating a floater in an eye comprises generating, by a scanning laser ophthalmoscope (SLO) device, an SLO image of the floater shadow cast by the floater onto the retina of the eye. The xy-location of the floater shadow, where the xy-location is related to an xy-scanner, is provided by the SLO device. The z-location of the floater, where the z-location is relative to the retina, is provided by an interferometer device. A laser beam is generated by a laser device. The focal point of the laser beam is focused onto the z-location of the floater by a z-focusing component of the laser device. An SLO beam is received by the xy-scanner from the SLO device and directed along an SLO beam path towards the xy-location of the floater shadow. The laser beam is received by the xy-scanner from the laser device and directed along the SLO beam path towards the xy-location of the floater shadow.

Embodiments may include none, one, some, or all of the following features:
* The interferometer device comprises an optical coherence tomography (OCT) device.
* The interferometer device comprises a swept source A-scan interferometer (SSASI) device.
* The angular position of the xy-scanner, where the angular position corresponds to an xy-location expressed in encoder units, is detected by an xy-encoder. The xy-location expressed in encoder units is reported by the xy-encoder.
* Image processing of the SLO image of the floater shadow cast onto the retina is performed by the computer. The floater shadow is evaluated to determine if the floater can cause a visual disturbance. Results of the evaluation are output by the computer. The floater shadow may be evaluated by determining if the floater shadow is cast onto a foveal region or a parafoveal region of the eye or by determining if the floater shadow is larger than a critical shadow size.
* A report is generated by the computer. The report includes the SLO image of the floater shadow cast onto the retina, and educational information about the floater or a recommended treatment for the floater.

### BRIEF DESCRIPTION OF THE DRAWINGS

FIGURE 1 illustrates an example of an ophthalmic laser surgical system that may be used to treat a floater in an eye, according to certain embodiments;
FIGURE 2 illustrates an example of a retinal image that may be generated by the system of FIGURE 1;
FIGURE 3 illustrates an example of an SLO device that may be used in the system of FIGURE 1;
FIGURE 4A illustrates examples of a depth measurement that may be performed by an interferometer device;
FIGURE 4B illustrates an example of multiple A-scans forming a B-scan;
FIGURE 5 illustrates an example of interferometer device that may be used in the system of FIGURE 1;
FIGURE 6 is a graph illustrating an example of tracking and predicting the xy-location of a floater shadow, which may be performed by the system of FIGURE 1, according to certain embodiments;
FIGURE 7 illustrates an example of a method for treating a floater in an eye, which may be performed by the system of FIGURE 1, according to certain embodiments; and
FIGURE 8 illustrates an example of a method for evaluating floaters, which may be performed by the system of FIGURE 1, according to certain embodiments.

### DESCRIPTION OF EXAMPLE EMBODIMENTS

Referring now to the description and drawings, example embodiments of the disclosed apparatuses, systems, and methods are shown in detail. The description and drawings are not intended to be exhaustive or otherwise limit the claims to the specific embodiments shown in the drawings and disclosed in the description. Although the drawings represent possible embodiments, the drawings are not necessarily to scale and certain features may be simplified, exaggerated, removed, or partially sectioned to better illustrate the embodiments.

In laser vitreolysis surgery, a laser beam should be accurately and precisely directed at a floater in order to treat the floater safely and effectively. However, floaters are extremely difficult to visualize with known laser vitreolysis systems. Light reflected from the floaters and background typically does not yield images with enough contrast to clearly distinguish the floaters from the background. Thus, accurately determining the location of the floater is challenging. Moreover, known laser vitreolysis systems cannot provide satisfactory image guidance for the laser beam. In these systems, the imaging beam for generating images and the laser beam for treating the floater are often not aligned, resulting in inaccurate laser beam guidance.

Certain embodiments of ophthalmic laser surgical systems described herein address these problems. For example, a scanning laser ophthalmoscope (SLO) device generates an image of the retina that shows the floater shadow cast by a floater onto the retina. The floater shadow yields an image with a higher contrast, and thus can be used to gather accurate information about the size, density, location, and clinical significance of the floater.

As another example, the SLO device provides xy-locations in encoder units of an xy-scanner. The encoder units represent the angular orientation of the mirrors of the xy-scanner. Providing locations in encoder units is easier than converting encoder information into linear distances (e.g., millimeter distances) on the retina because the SLO beam propagates through several curved optical surfaces (e.g., surfaces of the cornea, natural lens, and/or intraocular lens).

As yet another example, embodiments include an interferometer device (e.g., a swept source full depth optical coherence tomography (SSFD OCT) devices or a swept source A-scan interferometer (SSASI) device) that provides the z-location of the floater relative to the retina. As yet another example, the treatment laser beam shares an xy-scanner with the SLO and interferometric beams, allowing the laser beam to co-propagate with the SLO and interferometric beams. Since the SLO, interferometric, and laser devices use the same xy-scanner, the floater can be treated with high spatial accuracy.

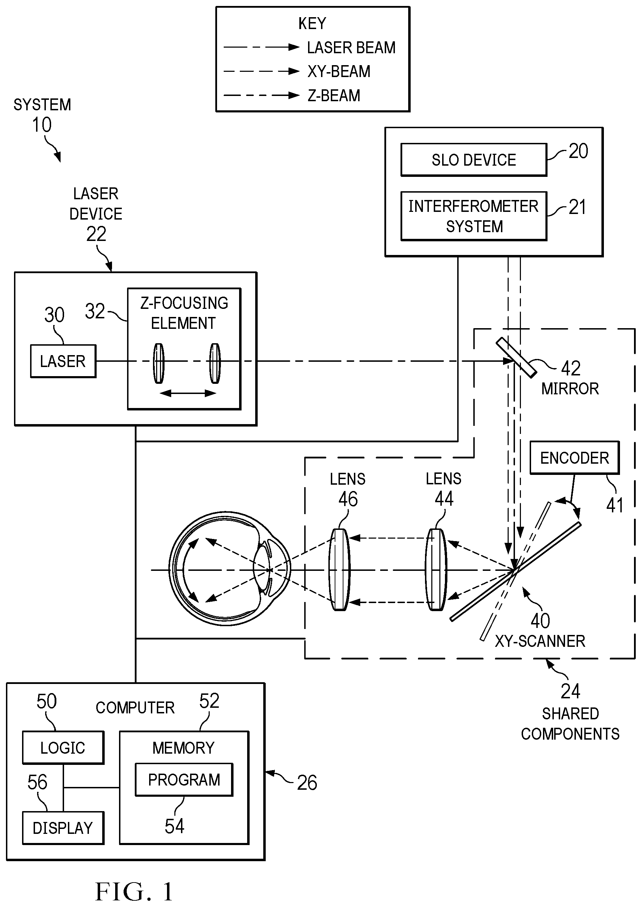

FIGURE 1 illustrates an example of an ophthalmic laser surgical system 10 that may be used to treat a floater in an eye, according to certain embodiments. As an overview, system 10 includes a scanning laser ophthalmoscope (SLO) device 20, an interferometer device 21, a laser device 22, one or more shared components 24, and a computer 26, coupled as shown. Laser device 22 includes an ultrashort pulse laser 30 and a z-focusing component 32, coupled as shown. Shared components 24 include an xy-scanner 40, an xy-encoder 41, and optical elements (such as a mirror 42 and lenses 44 and 46), coupled as shown. Computer 26 includes logic 50, a memory 52 (which stores a computer program 54), and a display 56, coupled as shown.

As an overview of operation of system 10, SLO device 20 generates an SLO image of the floater shadow cast by a floater onto the retina. SLO device 20 also provides the xy-location of the floater shadow, where the xy-location is related to xy-scanner 40. Interferometer device 21 provides the z-distance of the floater from the retina (which may be referred to as the z-location). Z-focusing component 32 of laser device 22 receives the z-location of the floater from interferometer device 21 and is designed to focus the focal point of the laser beam onto the z-location of the floater. Xy-scanner 40 receives an SLO beam from the SLO device and in response to a command from computer 26 can direct the SLO beam along an SLO beam path towards the xy-location of the floater shadow. Xy-scanner 40 also receives the laser beam from the laser device and directs the laser beam along the SLO beam path towards the xy-location of the floater shadow.

As an example of aiming the laser beam, an image of the eye includes a reticle, which is a graphical overlay (e.g., crosshairs) that indicates where the beam is currently aimed in the xy-plane. The user or computer 26 places the reticle over the floater shadow in the image to aim the beam at the floater. Xy-encoder 41 detects the position of xy-scanner 40 to determine the xy-location of the reticle (in encoder units) centered at the floater shadow.

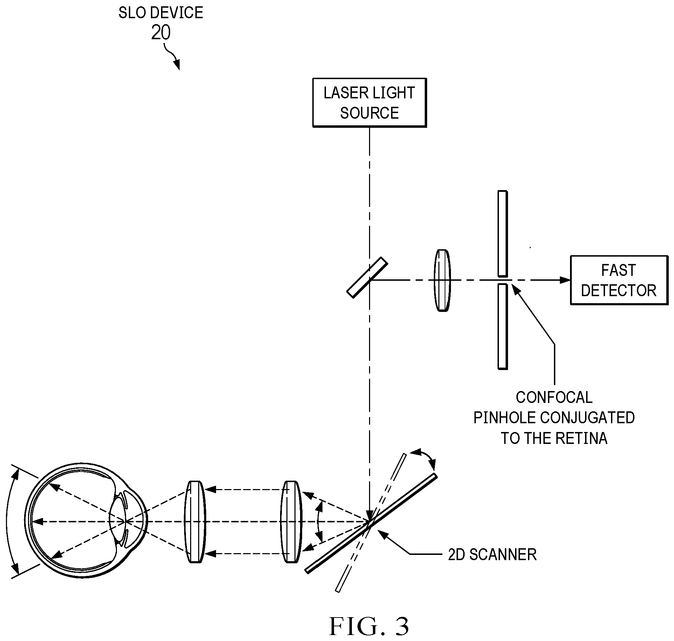

Turning to the parts of the system, SLO device 20 utilizes confocal laser scanning to generate images of the interior of the eye. In certain embodiments, SLO device 20 generates an image of the floater shadow that a floater casts on the retina and provides the xy-location of the floater shadow in encoder units. An example of SLO device 20 is described in more detail with reference to FIGURE 3.

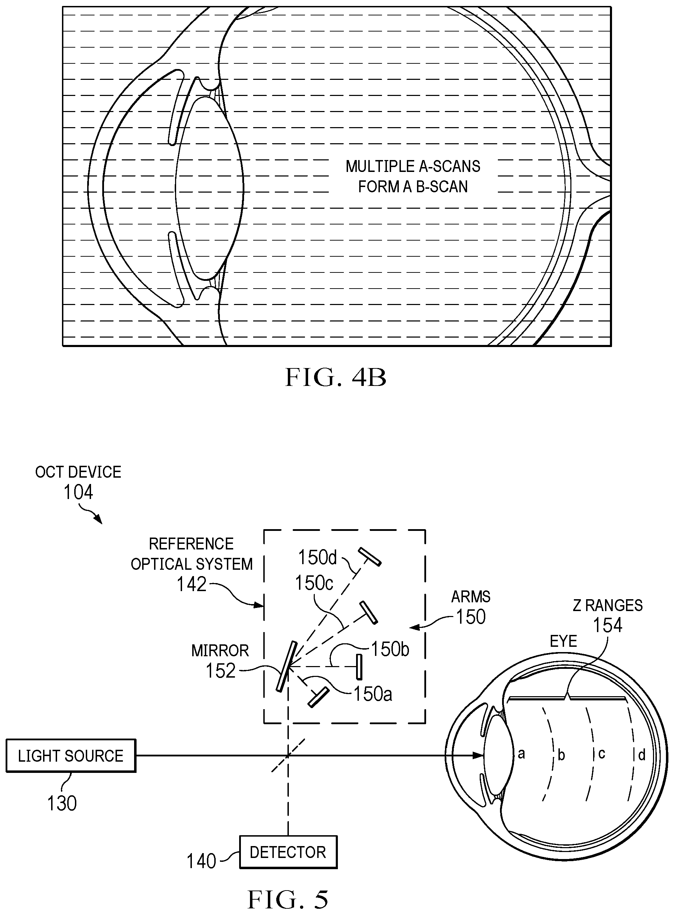

Interferometer device 21 provides the z-location of the floater relative to the retina. Interferometer device 21 has any suitable interferometer, e.g., a Fourier domain type (such as a swept source or a spectral domain type) that utilizes a fast Fourier transform (FFT). Examples of interferometer device 21 include an optical coherence tomography (OCT) device (such as a swept-source OCT device) and a swept source A-scan interferometer (SSASI) device (where a SASSI device performs only A-scans). Swept Source OCT and SSASI devices have a measuring range up to about 30 millimeters (mm) that can measure the depth (i.e., z-location relative to the retina) within the full length of the eye from the cornea to the retina. An example of an interferometer device 21 with multiple reference arms is described in more detail with reference to FIGURE 5.

As an example of operation of interferometer device 21, a splitter splits the light into measurement light and reference light. The reference light is directed to a reference arm system. The measurement light is directed through shared components 24 towards the eye and is reflected by surfaces and/or objects within the eye (e.g., the anterior and posterior surfaces of the cornea and natural or intraocular lens, the retina, and floaters). An interferometer combines the reflected measurement and reference light, which creates interference that in turn causes spectral modulation of the intensity. The frequency of the modulation is used to determine the depth at which the light was reflected, and the amplitude of the modulation carries information about the intensity of the back-reflected beam. The calculations may involve, e.g., Fourier analysis.

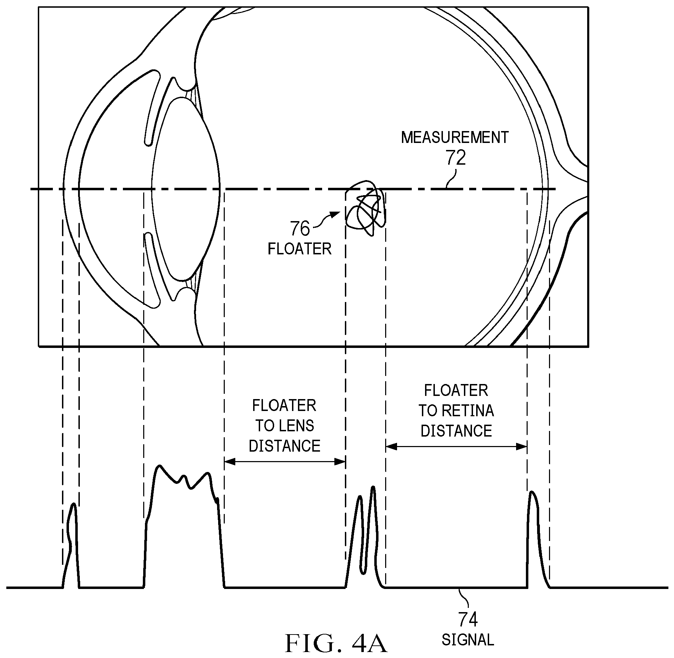

A measurement made along one direction of the xy-scanner is an A-scan. An example of an A-scan is described in more detail with reference to FIGURE 4A. Multiple A-scans made with the movement of a scanner mirror of the xy-scanner is a B-scan. A B-scan may be used to visualize a side view of a slice of the eye. An example of a B-scan is described in more detail with reference to FIGURE 4B. Multiple B-scans may be used to generate a 3D image of the eye.

The OCT images may be used to identify the location of the surfaces (e.g., the anterior and/or posterior surfaces) of a floater, the lens (natural or intraocular lens (IOL)), and/or the retina. Accordingly, the OCT images can indicate the z-location and thickness of the floater in the z-direction.

Turning to laser device 22, ultrashort pulse laser 30 generates a laser beam with any suitable wavelength, e.g., in a range from 400 nm to 2000 nm. Laser device 22 delivers laser pulses at any suitable repetition rate (e.g., a single pulse to 200 megahertz (MHz)). A laser pulse has any suitable pulse duration (e.g., 20 femtoseconds (fs) to 1000 nanoseconds (ns)), any suitable pulse energy (e.g., 1 nanojoule (nJ) to 10 millijoule (mJ)), and a focal point of any suitable size (e.g., 1 to 30 microns (µm)). In a particular embodiment, the laser is a picosecond or femtosecond laser with a repetition rate that exceeds 100 pulses per second (pps).

In certain embodiments, laser device 22 or the optical delivery system includes adaptive optics. The adaptive optics correct phase front errors of the laser beam to minimize the spot size of the laser beam, which in turn minimizes the required pulse energy (e.g., a few microjoules (uJ) to the nanojoule (nJ) range) and radiation exposure at the retina. In certain embodiments, adaptive optics are used to optimize the laser beam prior to treatment. In the embodiments, the laser beam is directed near the floater using subthreshold energy levels. A feedback signal (e.g., a two-photon fluorescence or a second harmonic feedback signal) from the vitreous is detected. Adaptive optics (e.g., an adaptive mirror) in the laser beam path are used to maximize the intensity of the feedback signal to minimize aberrations of the eye and the optical system.

In certain embodiments, laser device 22 includes an optical element that forms a Bessel or Bessel-like long focal length beam, which may increase the efficiency of floater destruction. In general, as compared with Gaussian beams, Bessel beams have a 1.6x smaller spot size, longer focal length (resulting in shorter treatment time), and larger divergence (yielding a larger spot size on the retina, reducing risk of retinal damage). Examples of optical elements that form Bessel or Bessel-like long focal length beams include an axicon, circular grating, proper phase plate, spatial light modulator (SLM), and Fabry-Perot interferometer.

Z-focusing component 32 longitudinally directs the focal point of the laser beam to a specific location in the direction of the floater shadow. In certain embodiments, z-focusing component 32 receives the z-location of the floater from interferometer device 21 (and may receive it via computer 26), and directs the laser beam towards the z-location of the floater. Z-focusing component 32 may include a lens of variable refractive power, a mechanically tunable lens, an electrically tunable lens (e.g., Optotune lens), an electrically or mechanically tunable telescope. In certain embodiments, laser device 22 or the optical delivery system also includes a fast xy-scanner used in tandem with z-focusing component 32 to, e.g., create a 3D focal spot pattern. Examples of such scanners include galvo, MEMS, resonant, or acousto-optical scanners.

Shared components 24 direct beams from SLO device 20, interferometer device 21, and laser device 22, respectively, towards the eye. Because SLO, interferometer, and/or laser beams share components 24, the beams are affected by the same optical distortions (e.g., fan distortion of scanners, barrel or pillow distortions of the scanner lens, refractive distortions from the inner eye surfaces, and other distortions). The distortions affect the beams in the same way, so the beams propagate along the same path. This allows for aiming the laser beam precisely at the floater.

As an overview of operation of shared components 24, mirror 42 directs a beam (SLO, interferometer, and/or laser beam) towards xy-scanner 40, which transversely directs the beam towards lens 44. Lenses 44 and 46 direct the beam towards eye. Shared components 24 may also provide spectral and polarization coupling and decoupling of SLO, interferometer, and laser beams to allow the beams to share the same path.

Turning to the details of shared components 24, in certain embodiments, xy-scanner 40 receives the xy-location of the floater shadow from SLO device 20, and directs the SLO, interferometer, and/or laser beam towards the xy-location. Xy-scanner 40 may be any suitable xy-scanner that transversely directs the focal point of the beam in the x- and y-directions and changes the angle of incidence of the beam into the pupil. For example, xy-scanner 40 includes a pair of galvanometrically-actuated scanner mirrors that can be tilted about mutually perpendicular axes. As another example, xy-scanner 40 includes an acousto-optical crystal that can acousto-optically steer the beam. As another example, xy-scanner 40 includes a fast scanner (e.g., a galvo, resonant, or acousto optical scanner) that can create, e.g., a 2D matrix of laser spots.

Xy-encoder 41 detects the angular position of xy-scanner 40 and reports the position as the xy-location measured in angular units. For example, xy-encoder 41 detects the angular orientations of the galvanometer mirrors of xy-scanner 40 in encoder units. Xy-encoder 41 may report the position in encoder units to SLO device 20, interferometer device 21, laser device 22, and/or computer 26. Since SLO device 20, interferometer device 21, and laser device 22 share xy-scanner 40, computer 26 can use the encoder units to instruct system 20 and device 22 where to aim their beams, making it unnecessary to perform the computer-intensive conversion from encoder units to a length unit such as millimeters. Xy-encoder 41 reports the positions at any suitable rate, e.g., once every 5 to 50 milliseconds (ms), such as every 10 to 30 or approximately every 20 ms.

Shared components 24 also include optical elements. In general, an optical element can act on (e.g., transmit, reflect, refract, diffract, collimate, condition, shape, focus, modulate, and/or otherwise act on) a laser beam. Examples of optical elements include a lens, prism, mirror, diffractive optical element (DOE), holographic optical element (HOE), and spatial light modulator (SLM). In the example, optical elements include mirror 42 and lenses 44 and 46. Mirror 42 may be a trichroic mirror. Lenses 44 and 46 may be scanning optics of an SLO device.

Computer 26 controls components of system 10 (e.g., SLO device 20, interferometer device 21, laser device 24, and/or shared components 24) in accordance with a computer program 54. Computer 26 may be separated from components or may be distributed among system 10 in any suitable manner, e.g., within SLO device 20, interferometer device 21, laser device 24, and/or shared components 24. In certain embodiments, portions of computer 26 that control SLO device 20, interferometer device 21, laser device 24, and/or shared components 24 may be part of SLO device 20, interferometer device 21, laser device 24, and/or shared components 24, respectively.

Computer 26 controls the components of system 10 in accordance with a computer program 54. Examples of computer programs 54 include floater shadow imaging, floater shadow tracking, image processing, floater evaluation, retinal exposure calculation, patient education, and insurance authorization programs. For example, computer 26 uses a computer program 54 to instruct SLO device 20, interferometer device 21, laser device 24, and/or shared components 24 to image a floater shadow and focus a laser beam at the floater.

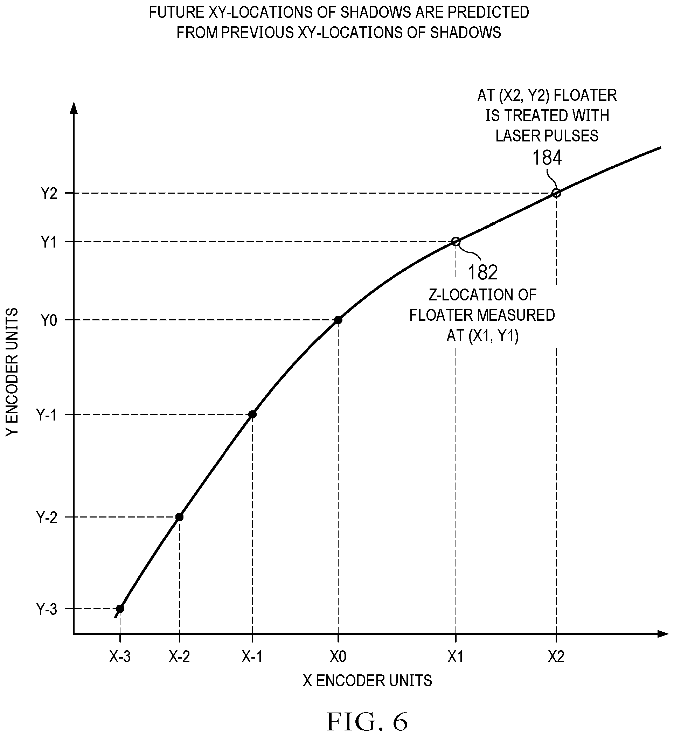

In certain embodiments, computer 26 uses an image processing program 54 to analyze the digital information of the image to extract information from the image. In certain embodiments, image processing program 54 analyzes an image of a floater shadow to obtain information about the floater. For example, program 54 detects a floater by detecting a darker shape in an image (using, e.g., edge detection or pixel analysis) that may be the floater shadow. As another example, program 54 detects the shape and size of a floater shadow, which indicate the size and shape of the floater. As another example, program 54 detects the tone or luminance of the floater shadow, which indicates the density of the floater. In certain embodiments, computer 26 uses a tracking program 54 to track a floater shadow, as described in more detail with reference to FIGURE 6.

In certain embodiments, computer 26 determines the radiant exposure at the retina from a laser pulse directed at a particular z-location. The determination may consider any suitable factors, e.g., laser pulse energy, laser radiation wavelength, number of laser pulses, laser pulse duration, cone angle of the focused laser beam, and the focus to the retina. For example, the exposure can be calculated using the laser spot size of the laser beam and the distance between the floater and retina. The radiant exposure should be less than a maximum radiant exposure, which may be determined in accordance with accepted standards. For example, the maximum radiant exposure may be set in accordance with ANSI Z80.36-2016. If the radiant exposure exceeds the maximum radiant exposure of the retina, lens, and/or IOL, computer 26 may modify any suitable factor (e.g., lower the pulse energy), provide a notification to the user, and/or prevent firing of the laser beam as an important safety feature.

System 10 may be used as a diagnostic tool and/or a treatment device, which can save space in an ophthalmic office. In certain embodiments, system 10 can be used as a diagnostic tool. In the embodiments, the laser is not activated, and system 10 can display images of the floater shadows, which can help many people over the age of 50 who have vitreous floaters. In most cases, floaters do not affect the visual acuity or visual performance of the patients. However, moving floaters attract the visual attention of the patients, annoying them. Showing images of floater shadows moving on the fovea to patients and explaining that floaters do not cause blindness and the visual effects are similar to that in movie theaters may calm down many patients. The patients may decide to not to treat the floaters, but accept them as an age-related benign condition.

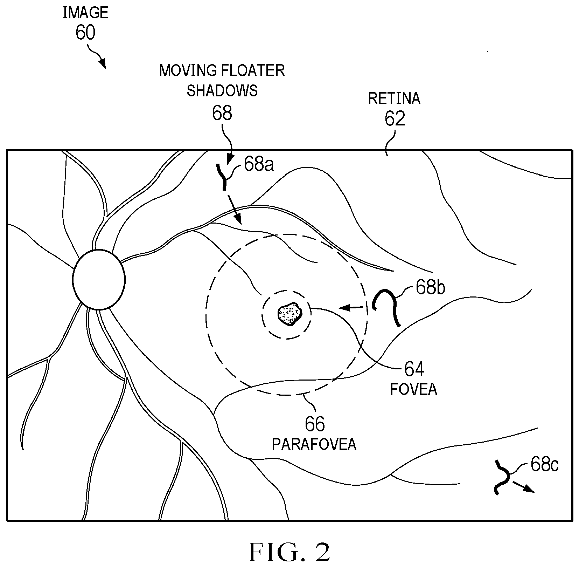

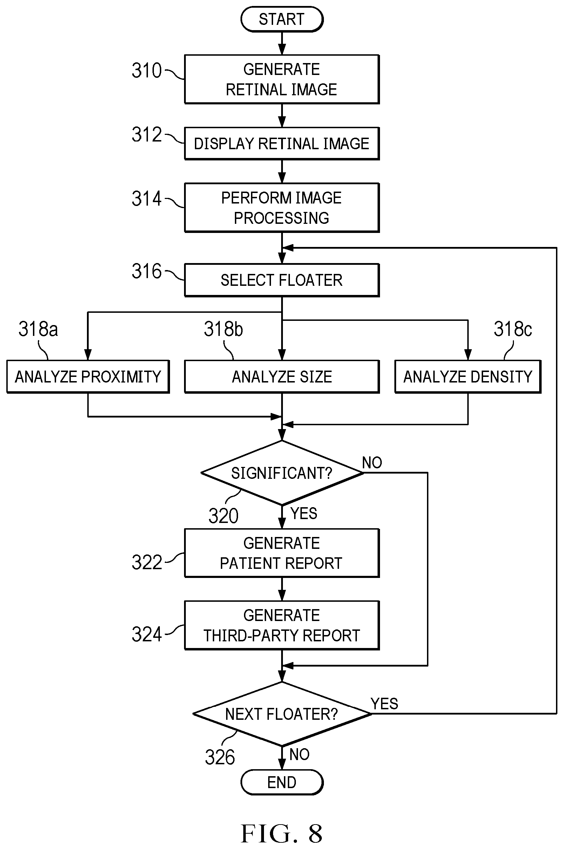

In the embodiments where system 10 is used as a diagnostic tool, certain computer programs may be appropriate. In certain embodiments, computer 26 uses a floater evaluation and diagnosis program 54 to evaluate a floater to determine if the floater is clinically significant, i.e., affects vision. In certain embodiments, display 56 of computer 26 displays images (such as a video) of a floater shadow so a user can evaluate the floater as described in more detail with reference to FIGURES 2 and 8. In other embodiments, computer 26 uses image processing to evaluate the floater shadow as described in more detail with reference to FIGURES 2 and 8.

In certain embodiments, a patient education program 54 generates a patient education report describing floater shadows found within a patient's eye. The report may include, e.g., images (such as a video) of a floater shadow found within a patent's eye; educational information about vitreous floaters; images of the vitreous pre- and post-treatment show the effectiveness of treatment; and/or other information to be provided to a patient. Computer 26 may output the report in any suitable manner. For example, computer 26 may store the report in memory 52, display the report in display 56, or send the report to, e.g., the user or patient.

In certain embodiments, a health insurance authorization program 54 generates an authorization report to obtain approval to treat floaters found within a patient's eye. The report may include, e.g., images (such as a video) of a floater shadow found within a patent's eye; patient information (e.g., identifying information, medical records); a recommended treatment; and/or other information required to obtain approval for treatment. Computer 26 may output the report in any suitable manner. For example, computer 26 may store the report in memory 52, display the report in display 56, or send the report to, e.g., the user, patient, or insurance company.

Involuntary and voluntary eye movements (e.g., saccadic and microsaccadic movements, drift, and tremor) can make laser treatment difficult. To reduce movement, the eye can be stabilized during treatment in any suitable manner to reduce movement of the eye. For example, the treated eye and/or the other eye can be stabilized using a fixation light. As another example, a patient interface or handheld surgical contact lens can be used to mechanically stabilize the eye. In addition, movement of the treated eye and/or the other eye can be tracked in any suitable manner. Any suitable portion of the eye (e.g., pupil, pupil edge, iris, blood vessels) and/or reflections from the eye (e.g., Purkinje reflections) can be tracked.

FIGURE 2 illustrates an example of a retinal image 60 that may be generated by system 10 of FIGURE 1. Image 60 shows the retina 62 of an eye, with a foveal region (or fovea) 64 and a parafoveal region (or parafovea) 66. Generally, fovea 64 has a visual angle of approximately +/- one degree, and parafovea 66 has a visual angle of approximately +/- seven degrees. Image 60 also shows floater shadows 68 (68a, 68b, 68c) that floaters cast on retina 62. In general, non-moving shadows are not caused by floaters, and may be caused by, e.g., corneal or lens opacities or anatomical changes of the retina, so floater treatment is not concerned with non-moving shadows.

A floater may be regarded as clinically significant if it can cause a visual disturbance, which can be determined from any suitable features of the floater shadow, e.g., the size and/or density of the shadow, proximity of the shadow to the fovea and/or parafovea, and/or the track of the shadow relative to the fovea and/or parafovea. As an example, a floater can cause a visual disturbance if it permanently or transiently casts a shadow 68 on fovea 64 or can cause distraction or annoyance if it permanently or transiently casts a shadow 68 on parafovea 66. Accordingly, if a floater shadow falls within or is predicted to move within fovea 64 and/or parafovea 66, the floater may be designated as clinically significant. As another example, floater shadow 68 can be used to estimate the size and density of the floater. Larger, denser floaters are more likely to cause a visual disturbance. Thus, a shadow 68 larger than a critical shadow size can indicate a clinically significant floater. A shadow 68 with a higher contrast relative to the background may indicate a clinically significant floater.

In some cases, a clinically significant floater may not be in a position to be safely treated. For example, floater shadow 68 may be too close to fovea 64, large blood vessels, the optic nerve head, or other sensitive area to be treated. In certain embodiments, computer 26 uses image processing to determine if a floater is in a position to be safely treated, and provides a notification if it is not, as described in more detail with reference to FIGURE 1.

In certain embodiments, a user such as a surgeon may determine significance from the displayed images (such as a video) of the floater shadow. An image processing program can assist the user in making the decision. In other embodiments, the computer can use image processing and target evaluation computer programs to determine significance from the image, as described in more detail with reference to FIGURE 8.

FIGURE 3 illustrates an example of SLO device 20 that may be used in system 10 of FIGURE 1. In an example of operation, laser beam is focused onto the retina and scanned over an angular range (e.g., a 20 to 40 degree angular range) by a 2D xy-scanner (e.g., a galvo scanner). The light reflected from the retina is focused by a lens onto a pinhole. The pinhole is optically conjugated to the retinal surface such that only the light reflected from the retina is detected by a detector (e.g., a sensitive high-speed detector) and the other light is filtered out. The intensity of the back-reflected light is displayed as a 2D enface image, where the x- and y- axes of the image represent readings of an angular encoder of the xy-scanner. The xy-scanner and detector may be sufficiently fast to display the enface image as a video with any suitable frame rate (e.g., up to about 100 frames per second).

The SLO image displays the local intensity of the back-reflected light from the retina, which shows the anatomical features of the retina (e.g., vasculature, optic nerve head, and certain retinal decease). The image also shows shadows cast by floaters. Floaters are opaque objects that attenuate an incident laser beam, causing shadows on the retina. Floaters move with the partially liquified vitreous, so they cause moving shadows. The movement distinguishes floater shadows from static images of, e.g., anatomical objects of the retina or other parts of the eye.

FIGURE 4A illustrates examples of a depth measurement 72 that may be performed by an interferometer device, such as an optical coherence tomography (OCT) device (such as a swept-source OCT device) or a swept source A-scan interferometer (SSASI) device. Depth measurement 72 may comprise an A-scan that extends from the cornea through the lens and floater 76 to the retina. The signal 74 from the A-scan indicates reflections from the cornea, lens, floater, and retina, which can used to determine the z-locations of these features relative to the retina.

FIGURE 4B illustrates an example of multiple A-scans forming a B-scan. Multiple A-scans made with the movement of a scanner mirror of the xy-scanner (or other movement that yields a plane of A-scans) is a B-scan. A B-scan may be used to visualize a side view of a slice of the eye.

FIGURE 5 illustrates an example of interferometer device 21 that may be used in system 10 of FIGURE 1. Interferometer device 21 may be an optical coherence tomography (OCT) device (such as a swept-source OCT device) or a swept source A-scan interferometer (SSASI) device. In the example, interferometer device 21 includes reference optical system 142 (with arms 150 (150a to 150d) and mirror 152), light source 130, and detector 140, coupled as shown. Light source 130 provides light for the interferometer beam. Examples of light source 130 include a super-luminescent or swept-source diode, such as a super-luminescent diode. Detector 140 detects the interference signal light. Examples of detector 140 include a high-resolution spectrometer or fast diode.

Reference optical system 142 includes any suitable number of reference arms 150 (150a to 150d) and galvo mirror 152. Each reference arm 150 is used for a different z-range 154 of the eye. For example, arm 150a is used for z-range 154a, arm 150b is used for z-range 154b, arm 150c is used for z-range 154c, and arm 150d is used for z-range 154d. In certain embodiments, the z-ranges 154 may overlap slightly, e.g., 1 mm or less. In the example, each z-range 154 corresponds to approximately 6 mm of vitreous, yielding coverage of approximately 24 mm. Galvo mirror 152 is used to direct the beam to the arm 150 for a particular z-range 154, and may switch between arms in, e.g., less than 5 ms, such as approximately one ms. Floaters have limited movement in the z-direction, so once an arm 150 for a z-range is selected, there may be little need to switch to a different arm 150. Computer 26 can join together images from different z-ranges 154 to yield an image of the length of the eye. In certain embodiments, interferometric devices, such as a swept source OCT device or a swept source A-scan interferometer (SSASI) device, may have measurement range as large as 35 mm, so they do not need multiple reference arms.

FIGURE 6 is a graph 180 illustrating an example of tracking and predicting the xy-location of a floater shadow, which may be performed by system 10 of FIGURE 1, according to certain embodiments. In the embodiments, a computer uses a tracking program to track and/or predict the movement of a floater shadow. For example, the computer performs image analysis of retinal images to track the movement of the floater shadow to track the floater. As discussed with reference to FIGURE 2, floater treatment is concerned with moving shadows.

In the example, the xy-location is given in encoder units (which may be provided by encoder 41). Variable t represents time t = -3, -2, -1, 0, 1, 2, where t = 0 is the current time, t = -3, -2, -1 is the past time, and t = 0, 1, 2 is the future time. Yt represents the y-location in encoder units at time t, and Xt represents the x-location in encoder units at time. The tracking program predicts the future xy-locations by extrapolating from past xy-locations.

In the example, the interferometer device measures the z-location position of the floater at the xy-location (x1, y1) at time t = 1, shown at reference number 182. The measurement may be performed a few milliseconds prior to firing the laser. At the xy-location (x2, y2) at time t = 2, shown at reference number 184, the laser device fires a laser beam comprising laser pulses. The laser beam is directed by the xy-scanner to the xy-location (x2, y2) and is focused by the z-focusing component at the z-location. In general, floaters do not move much in the z-direction, so the z-location measured at t = 1 may be sufficiently close to the z-location at t = 2.

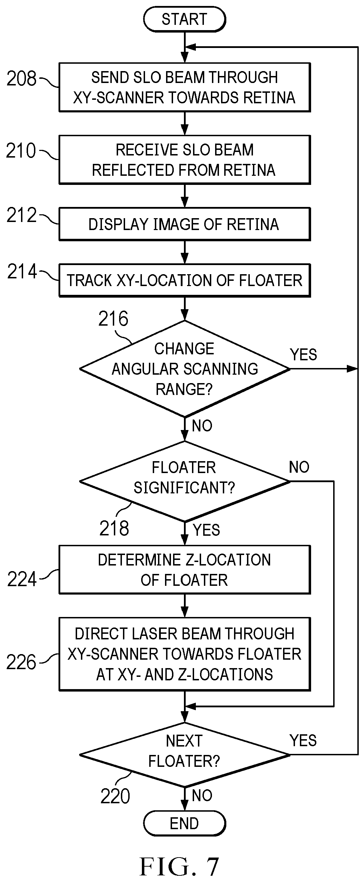

FIGURE 7 illustrates an example of a method for treating a floater in an eye, which may be performed by system 10 of FIGURE 1, according to certain embodiments. The method starts at step 208, where an SLO device sends an SLO beam through an xy-scanner towards the eye. During an initial viewing, the SLO device scans a larger angular range in order to identify a region with a floater shadow. For example, the larger angular range may be 20 to 60 degrees, such as 30 to 50 degrees, e.g., approximately 40 degrees, with a frame rate of, e.g., over 40 or over 50 frames/s. The SLO device receives the SLO beam reflected from the retina at step 210. In the SLO device, the reflected light goes through a confocal filter that transmits light from the retina and blocks the other reflected light. A display displays images (e.g., a video) at step 212 that show the floater shadow on the retina.

The floater shadow is tracked at step 214 using the SLO image to determine the xy-location of the shadow, as described in more detail with reference to FIGURE 6. The angular scanning range may be changed at step 216. The image may show a region with the floater shadow, and a smaller angular range (e.g., be 2 to 10 degrees, such as approximately 5 degrees) may be used to narrow the angular scanning to that region. Since the angular scanning range is smaller, a higher frame rate may be used, e.g., over 100 frames/s. If the angular scanning is to be changed at step 216, the method returns to steps 210 and 214 to scan the different angular range and display the resulting image. If the angular scanning range is not to be changed at step 216, the method proceeds to step 218 to determine whether the floater is clinically significant.

The floater may be clinically significant, i.e., may be expected to cause a visual disturbance, at step 218. The floater shadow may be evaluated to determine the clinical significance of the floater, as described in more detail with reference to FIGURES 2 and 8. If the floater is not clinically significant at step 218, the method proceeds to step 220 to check the next floater shadow. If the floater is clinically significant at step 218, the method proceeds to step 224.

The interferometer device determines the z-location and sends the z-location to the laser device at step 224, so that the laser device can aim the focal point of the laser beam at the z-location, as described in more detail with reference to FIGURE 6. The interferometer device may aim the interferometer beam at the xy-location of the floater shadow tracked by a tracking computer program to perform an A-scan in this direction. For example, a SSASI device may perform an A-scan in this direction, or an OCT device may perform A-scans in a small angular range (e.g., +/- two degrees) centered in this direction. The interferometer device may perform any suitable number of scans, e.g., a value in a range of 1 to 100, or 5 to 50, or 10 to 20 scans. In certain embodiments, the computer determines whether the expected radiance energy at the retina from a laser beam aimed at the z-location is safe, as described in more detail with reference to FIGURE 1.

Via steps 212 and 224, the surgical system determines the xy-location of the floater shadow and z-location of the floater, as described in more detail with reference to FIGURE 6. In certain embodiments, the xy-location is the location predicted for when the laser fires. The laser device directs the laser beam through the xy-scanner to the z-location of the floater at step 226. The xy-scanner directs the laser beam to the xy-location determined by the tracking at step 222. The method then proceeds to step 220 to check whether there is a next floater shadow. If there is a next floater shadow, the method returns to step 210, where the SLO device sends the SLO beam through the xy-scanner towards the next floater shadow. If there is no next floater shadow, the method ends.

In certain embodiments, laser device 22 is optimized prior to treatment. In the embodiments, the laser beam is directed near the floater using subthreshold energy levels. A feedback signal (e.g., a two-photon fluorescence or a second harmonic feedback signal) from the vitreous is detected. Adaptive optics (e.g., an adaptive mirror) in the laser beam path are used to maximize the intensity of the feedback signal to minimize aberrations of the eye and the optical system.

FIGURE 8 illustrates an example of a method for evaluating floaters, which may be performed by system 10 of FIGURE 1, according to certain embodiments. In the example, the method generates a retinal image of an eye and analyzes floater shadows of the image to evaluate floaters in the eye. The method starts at step 310, where an SLO device generates a retinal image of an eye. A computer displays the image on a display at step 312. The computer performs image processing on the image at step 314. An image processing program may detect the edges of a floater shadow indicating the presence of a floater. In certain embodiments, the computer overlays a graphical element onto the image that point out the shadow to a user, e.g., a circle that surrounds the shadow, an arrow that points to the shadow, or a border that outlines the shadow.

A shadow is selected to be analyzed at step 316. The user or the computer may select the shadow. Steps 318a, 318b, and 318c describe analyzing the shadow to determine if the floater is clinically significant, as described in more detail with respect to FIGURE 2. The proximity of the shadow to the fovea or parafovea is analyzed at step 318a. For example, a shadow on or passing over the fovea or parafovea may indicate a clinically significant floater. The size of the shadow is analyzed at step 318b. For example, a shadow larger than a critical size may indicate a clinically significant floater. The density of the shadow is analyzed at step 318b. For example, a shadow with a higher contrast relative to the background may indicate a clinically significant floater.

The method may include steps 318a, 318b, and/or 318c, i.e., the method need not include all the steps. In addition, the user and/or computer performs any or all of the included steps. In some cases, the user may decide to perform one or more steps, and then instruct the computer to perform other steps. Also, if the outcome of one step indicates that a floater is significant, the method may omit the remaining steps. For example, if steps 318a and 318b indicate the floater shadow is in the foveal region and is critically large, the method may omit step 318c. Alternatively, if the outcome of one step indicates that a floater is not significant, the method may omit the remaining steps. For example, if step 318a indicates the floater shadow is far from the parafoveal region, the method may omit steps 318b and 318c.

The floater may be designated as clinically significant at step 320 in response to the analyses performed at steps 318a, 318b, and/or 318c. The user and/or computer may make this decision. If the floater is significant, the method proceeds to steps 322 and/or 324, where reports are generated, as described in more detail with reference to FIGURE 1. The computer generates a patient report at step 322. For example, the patient report can be used to educate the patient about their condition. The computer generates an authorization report at step 324. For example, the authorization report can be used to obtain approval for treatment. In other embodiments of the method, the laser device may treat the floater, as described in more detail with respect to FIGURES 6 and 7, in addition to or as an alternative to generating reports. If the floater is not significant, the method proceeds directly to step 326.

There may be a next shadow in the image at step 326. If there is a next shadow, the method returns to step 316 to select the next shadow. If there are no more shadows, the method ends.

A component (such as the control computer) of the systems and apparatuses disclosed herein may include an interface, logic, and/or memory, any of which may include computer hardware and/or software. An interface can receive input to the component and/or send output from the component, and is typically used to exchange information between, e.g., software, hardware, peripheral devices, users, and combinations of these. A user interface is a type of interface that a user can utilize to communicate with (e.g., send input to and/or receive output from) a computer. Examples of user interfaces include a display, Graphical User Interface (GUI), touchscreen, keyboard, mouse, gesture sensor, microphone, and speakers.

Logic can perform operations of the component. Logic may include one or more electronic devices that process data, e.g., execute instructions to generate output from input. Examples of such an electronic device include a computer, processor, microprocessor (e.g., a Central Processing Unit (CPU)), and computer chip. Logic may include computer software that encodes instructions capable of being executed by an electronic device to perform operations. Examples of computer software include a computer program, application, and operating system.

A memory can store information and may comprise tangible, computer-readable, and/or computer-executable storage medium. Examples of memory include computer memory (e.g., Random Access Memory (RAM) or Read Only Memory (ROM)), mass storage media (e.g., a hard disk), removable storage media (e.g., a Compact Disk (CD) or Digital Video or Versatile Disk (DVD)), database, network storage (e.g., a server), and/or other computer-readable media. Particular embodiments may be directed to memory encoded with computer software.

Although this disclosure has been described in terms of certain embodiments, modifications (such as changes, substitutions, additions, omissions, and/or other modifications) of the embodiments will be apparent to those skilled in the art. Accordingly, modifications may be made to the embodiments without departing from the scope of the invention. For example, modifications may be made to the systems and apparatuses disclosed herein. The components of the systems and apparatuses may be integrated or separated, or the operations of the systems and apparatuses may be performed by more, fewer, or other components, as apparent to those skilled in the art. As another example, modifications may be made to the methods disclosed herein. The methods may include more, fewer, or other steps, and the steps may be performed in any suitable order, as apparent to those skilled in the art.

To aid the Patent Office and readers in interpreting the claims, Applicants note that they do not intend any of the claims or claim elements to invoke 35 U.S.C. §112(f), unless the words "means for" or "step for" are explicitly used in the particular claim. Use of any other term (e.g., "mechanism," "module," "device," "unit," "component," "element," "member," "apparatus," "machine," "system," "processor," or "controller") within a claim is understood by the applicants to refer to structures known to those skilled in the relevant art and is not intended to invoke 35 U.S.C. §112(f).

## Claims

1. An ophthalmic laser surgical system (10) for treating a floater in an eye, comprising:
a scanning laser ophthalmoscope, SLO, device (20) configured to:
generate an SLO image of a floater shadow cast by the floater onto a retina of the eye; and
provide an xy-location of the floater shadow, the xy-location related to an xy-scanner;
an interferometer device (21) configured to provide a z-location of the floater, the z-location relative to the retina;
a laser device (22) configured to generate a laser beam, the laser device comprising a z-focusing component (32) configured to:
focus a focal point of the laser beam onto the z-location of the floater; and
an xy-scanner (40) configured to:
receive an SLO beam from the SLO device (20) and direct the SLO beam along an SLO beam path towards the xy-location of the floater shadow; and
receive the laser beam from the laser device and direct the laser beam along the SLO beam path towards the xy-location of the floater shadow.

2. The ophthalmic laser surgical system of Claim 1, the interferometer device (21) comprising an optical coherence tomography, OCT, device.

3. The ophthalmic laser surgical system of Claim 1, the interferometer device (21) comprising a swept source A-scan interferometer (SSASI) device.

4. The ophthalmic laser surgical system of Claim 1, the interferometer device (21) comprising a plurality of reference arms (150), each arm corresponding to a z-range of a plurality of z-ranges within the vitreous, a z-range relative to the retina.

5. The ophthalmic laser surgical system of Claim 1, further comprising an xy-encoder (41) configured to:
detect an angular position of the xy-scanner (40), the angular position corresponding to an xy-location expressed in encoder units; and
report the xy-location expressed in encoder units.

6. The ophthalmic laser surgical system of Claim 1, the laser device (22) comprising:
adaptive optics configured to minimize a spot size of the laser beam.

7. The ophthalmic laser surgical system of Claim 1, the laser device (22) comprising:
adaptive optics configured to maximize a feedback signal to optimize the laser beam.

8. The ophthalmic laser surgical system of Claim 1, the laser device (22) comprising:
an optical element configured to form the laser beam as a Bessel or Bessel-like long focal length beam.

9. The ophthalmic laser surgical system of Claim 1, further comprising a computer (26) configured to:
perform image processing on the SLO image of the floater shadow cast onto the retina;
evaluate the floater shadow to determine if the floater can cause a visual disturbance;
and
output results of the evaluation.

10. The ophthalmic laser surgical system of Claim 9, the computer (26) configured to evaluate the floater shadow to determine if the floater can cause a visual disturbance by:
determining if the floater shadow is cast onto a foveal region or a parafoveal region of the eye.

11. The ophthalmic laser surgical system of Claim 9, the computer (26) configured to evaluate the floater shadow to determine if the floater can cause a visual disturbance by:
determining if the floater shadow is larger than a critical shadow size.

12. The ophthalmic laser surgical system of Claim 9, the computer (26) further configured to:
generate a report comprising:
the SLO image of the floater shadow cast onto the retina; and
educational information about the floater or a recommended treatment for the floater; and
output the report.

## Patentansprüche

1. Ophthalmisches Laserchirurgiesystem (10) zur Behandlung eines Floaters in einem Auge, das umfasst:
eine Scanlaser-Ophthalmoskop-, SLO-, Vorrichtung(20), die ausgelegt ist zum:
Erzeugen eines SLO-Bildes eines Floater-Schattens, der durch den Floater auf eine Netzhaut des Auges geworfen wird; und
Bereitstellen einer xy-Position des Floater-Schattens, wobei die xy-Position auf einen xy-Scanner bezogen ist;
eine Interferometervorrichtung (21), die dazu ausgelegt ist, eine z-Position des Floaters bereitzustellen, wobei die z-Position relativ zur Netzhaut ist;
eine Laservorrichtung (22), die dazu ausgelegt ist, einen Laserstrahl zu erzeugen, wobei die Laservorrichtung eine z-fokussierende Komponente (32) umfasst, die ausgelegt ist zum:
Fokussieren eines Brennpunkts des Laserstrahls auf die z-Position des Floaters; und
einen xy-Scanner (40), der ausgelegt ist zum:
Empfangen eines SLO-Strahls von der SLO-Vorrichtung (20) und Richten des SLO-Strahls entlang des SLO-Strahlpfads auf die xy-Position des Floater-Schattens; und
Empfangen des Laserstrahls aus der Laservorrichtung und Richten des Laserstrahls entlang des SLO-Strahlpfads auf die xy-Position des Floater-Schattens.

2. Ophthalmisches Laserchirurgiesystem nach Anspruch 1, wobei die Interferometervorrichtung (21) eine optische Kohärenztomographie-, OCT-, Vorrichtung umfasst.

3. Ophthalmisches Laserchirurgiesystem nach Anspruch 1, wobei die Interferometervorrichtung (21) eine Swept-Source-A-Scan-Interferometer(SSASI)-Vorrichtung umfasst.

4. Ophthalmisches Laserchirurgiesystem nach Anspruch 1, wobei die Interferometervorrichtung (21) mehrere Referenzarme (150) umfasst, wobei jeder Arm einem z-Bereich mehrerer z-Bereiche innerhalb des Glaskörpers, einem z-Bereich relativ zur Netzhaut, entspricht.

5. Ophthalmisches Laserchirurgiesystem nach Anspruch 1, das ferner einen xy-Codierer (41) umfasst, der ausgelegt ist zum:
Detektieren einer Winkelposition des xy-Scanners (40), wobei die Winkelposition einer xy-Position entspricht, die in Codierereinheiten ausgedrückt ist; und
Melden der in Codierereinheiten ausgedrückten xy-Position.

6. Ophthalmisches Laserchirurgiesystem nach Anspruch 1, wobei die Laservorrichtung (22) umfasst:
eine adaptive Optik, die dazu ausgelegt ist, eine Punktgröße des Laserstrahls zu minimieren.

7. Ophthalmisches Laserchirurgiesystem nach Anspruch 1, wobei die Laservorrichtung (22) umfasst:
eine adaptive Optik, die dazu ausgelegt ist, ein Rückkopplungssignal zu maximieren, um den Laserstrahl zu optimieren.

8. Ophthalmisches Laserchirurgiesystem nach Anspruch 1, wobei die Laservorrichtung (22) umfasst:
ein optisches Element, das dazu ausgelegt ist, den Laserstrahl als Bessel- oder Bessel-artigen langbrennweitigen Strahl zu bilden.

9. Ophthalmisches Laserchirurgiesystem nach Anspruch 1, das ferner einen Computer (26) umfasst, der ausgelegt ist zum:
Durchführen einer Bildverarbeitung am SLO-Bild des Floater-Schattens, der auf die Netzhaut geworfen wird;
Bewerten des Floater-Schattens, um zu bestimmen, ob der Floater eine Sehstörung verursachen kann; und
Ausgeben von Ergebnissen der Auswertung.

10. Ophthalmisches Laserchirurgiesystem nach Anspruch 9, wobei der Computer (26) dazu ausgelegt ist, den Floater-Schatten auszuwerten, um zu bestimmen, ob der Floater eine Sehstörung verursachen kann, durch:
Bestimmen, ob der Floater-Schatten auf einen Fovealbereich oder einen Parafovealbereich des Auges geworfen wird.

11. Ophthalmisches Laserchirurgiesystem nach Anspruch 9, wobei der Computer (26) dazu ausgelegt ist, den Floater-Schatten auszuwerten, um zu bestimmen, ob der Floater eine Sehstörung verursachen kann, durch:
Bestimmen, ob der Floater-Schatten größer als eine kritische Schattengröße ist.

12. Ophthalmisches Laserchirurgiesystem nach Anspruch 9, wobei der Computer (26) ferner ausgelegt ist zum:
Erzeugen eines Berichts, der umfasst:
das SLO-Bild des Floater-Schattens, der auf die Netzhaut geworfen wird; und
Aufklärungsinformationen über den Floater oder eine empfohlene Behandlung für den Floater; und
Ausgeben des Berichts.

## Revendications

1. Système chirurgical ophtalmique à laser (10) destiné au traitement d'un corps flottant dans un œil, comprenant :
un dispositif d'ophtalmoscope à laser de balayage, SLO (20) configuré pour :
générer une image SLO d'une ombre de corps flottant projetée par le corps flottant sur une rétine de l'œil ; et
fournir un emplacement xy de l'ombre de corps flottant, l'emplacement xy étant associé à un scanner xy ;
un dispositif interférométrique (21) configuré pour fournir un emplacement z du corps flottant, l'emplacement z étant relatif à la rétine ;
un dispositif laser (22) configuré pour générer un faisceau laser, le dispositif laser comprenant un composant de focalisation en z (32) configuré pour :
focaliser un point focal du faisceau laser sur l'emplacement z du corps flottant ; et
un scanner xy (40) configuré pour :
recevoir un faisceau SLO en provenance du dispositif SLO (20) et diriger le faisceau SLO le long d'un trajet de faisceau SLO vers l'emplacement xy de l'ombre de corps flottant ; et
recevoir le faisceau laser en provenance du dispositif laser et diriger le faisceau laser le long du trajet du faisceau SLO vers l'emplacement xy de l'ombre de corps flottant.

2. Système chirurgical ophtalmique à laser selon la revendication 1, le dispositif interférométrique (21) comprenant un dispositif de tomographie à cohérence optique, OCT.

3. Système chirurgical ophtalmique à laser selon la revendication 1, le dispositif interférométrique (21) comprenant un dispositif interférométrique à balayage A à source balayée (SSASI).

4. Système chirurgical ophtalmique à laser selon la revendication 1, le dispositif interférométrique (21) comprenant une pluralité de bras de référence (150), chaque bras correspondant à une plage z d'une pluralité de plages z à l'intérieur du corps vitré, une plage z étant relative à la rétine.

5. Système chirurgical ophtalmique à laser selon la revendication 1, comprenant en outre un codeur xy (41) configuré pour :
détecter une position angulaire du scanner xy (40), la position angulaire correspondant à un emplacement xy exprimé en unités de codeur ; et
indiquer l'emplacement xy exprimé en unités de codeur.

6. Système chirurgical ophtalmique à laser selon la revendication 1, le dispositif laser (22) comprenant :
une optique adaptative configurée pour minimiser une taille d'un point du faisceau laser.

7. Système chirurgical ophtalmique à laser selon la revendication 1, le dispositif laser (22) comprenant :
une optique adaptative configurée pour maximiser un signal de rétroaction afin d'optimiser le faisceau laser.

8. Système chirurgical ophtalmique à laser selon la revendication 1, le dispositif laser (22) comprenant :
un élément optique configuré pour former le faisceau laser sous la forme d'un faisceau à longue distance focale de Bessel ou de type Bessel.

9. Système chirurgical ophtalmique à laser selon la revendication 1, comprenant en outre un ordinateur (26) configuré pour :
effectuer un traitement d'image sur l'image SLO de l'ombre de corps flottant projetée sur la rétine ;
évaluer l'ombre de corps flottant pour déterminer si le corps flottant peut causer une perturbation visuelle ; et
émettre en sortie des résultats de l'évaluation.

10. Système chirurgical ophtalmique à laser selon la revendication 9, l'ordinateur (26) étant configuré pour évaluer l'ombre de corps flottant afin de déterminer si le corps flottant peut provoquer une perturbation visuelle en :
déterminant si l'ombre de corps flottant est projetée sur une région fovéale ou parafovéale de l'œil.

11. Système chirurgical ophtalmique à laser selon la revendication 9, l'ordinateur (26) étant configuré pour évaluer l'ombre de corps flottant afin de déterminer si le corps flottant peut provoquer une perturbation visuelle en :
déterminant si l'ombre de corps flottant est plus grande qu'une taille d'ombre critique.

12. Système chirurgical ophtalmique à laser selon la revendication 9, l'ordinateur (26) étant en outre configuré pour :
générer un rapport comprenant :
l'image SLO de l'ombre de corps flottant projetée sur la rétine ; et
des informations éducatives sur le corps flottant ou un traitement recommandé pour le corps flottant ; et
générer le rapport.
